# EUROPÄISCHE PATENTSCHRIFT

(11) **EP 3 451 857 B1**
(45) Veröffentlichungstag und Bekanntmachung des Hinweises auf die Patenterteilung: **14.07.2021**
(21) Anmeldenummer: 16725776.5
(22) Anmeldetag: 05.05.2016
(51) Int. Cl.: A23L 27/20, A23L 27/00, A23G 4/06, A61Q 11/00

(54) **KÜHLSTOFFMISCHUNGEN**
COOLANT MIXTURES
MÉLANGES D'AGENTS RAFRAÎCHISSANTS

(43) Veröffentlichungstag der Anmeldung: 13.03.2019
(73) Patentinhaber: Symrise AG, 37603 Holzminden (DE)
(72) Erfinder: SIEGEL, Sven, 37671 Höxter (DE); MACHINEK, Arnold, 37603 Holzminden (DE); HÖLSCHER, Bernd, 37620 Halle (DE)
(74) Vertreter: Daniels, Stefanie Lisa
(86) Internationale Anmeldenummer: PCT/EP2016/060127
(87) Internationale Veröffentlichungsnummer: WO 2017/190789

(56) Entgegenhaltungen:
- EP-A2- 1 050 574
- WO-A2-2013/171018
- US-A1- 2011 318 459
- LAWRENCE B M: "Some new trace constituents in the oil of Mentha piperita L", ANAIS DA ACADEMIA BRASILEIRA DE CIENCIAS,, Bd. 44, 1. Januar 1972 (1972-01-01), Seiten 191-197, XP008031353, ISSN: 0001-3765
- SAMANT S S ET AL: "DIVERSITY, DISTRIBUTION AND INDIGENOUS USES OF ESSENTIAL OIL-YIELDING MEDICINAL PLANTS OF THE INDIAN HIMALAYAN REGION", CURRENT RESEARCH ON MEDICINAL AND AROMATIC PLANTS, CENTRAL INSTITUTE OF MEDICINAL AND AROMATIC PLANTS, LUCKNOW, IN, Bd. 22, Nr. 1B, 1. März 2000 (2000-03-01), Seiten 671-684, XP001094687, ISSN: 0253-7125
- DATABASE WPI Week 200867 Thomson Scientific, London, GB; AN 2008-L32543 XP002762606, & CN 101 156 572 A (JIANGXI SHANGFENG DAILY CHEM CO LTD) 9. April 2008 (2008-04-09)

## Beschreibung

### GEBIET DER ERFINDUNG

Die Erfindung befindet sich auf dem Gebiet oralen Zubereitungen und betrifft Kühlstoffmischungen mit verbesserten Produkteigenschaften sowie orale Zubereitungen, die diese Stoffe enthalten.

### STAND DER TECHNIK

Unter dem Begriff Kühlstoff versteht man chemische Verbindungen, die beim Kontakt mit Haut oder Schleimhaut einen kühlenden Sinneseindruck hinterlassen. Solche Stoffe finden in einer Vielzahl von kosmetischen Produkten Verwendung, ursprüngliches und wichtigstes Anwendungsgebiet sind aber Nahrungsmittel oder allgemeiner ausgedrückt, orale Zubereitungen. Diese umfassen auch Mund- und Zahnpflegemittel sowie bestimmte pharmazeutische Zubereitungen, insbesondere solche, die zur Linderung von Erkältungskrankheiten eingesetzt werden. Aufgabe der Kühlstoffe ist es, einen erfrischenden, aber auch beruhigenden und mitunter sogar schmerzlindernden Geschmackeindruck auf den Schleimhäuten zu bewirken. Unter Umständen wirken Kühlstoffe auch abschwellend.

Der bekannteste und bis heute immer noch sehr wichtige Kühlstoff ist das Menthol, ein monozyklischer Terpenalkohol, das wesentlicher Bestandteil des Minzöls ist. Seit den 60er Jahren des vergangenen Jahrhunderts sind die Kühlstoffe in mehreren Wellen durch eine Vielzahl von neuen Verbindungen mit unterschiedlichen Produkteigenschaften bereichert worden, so dass bei der Produktentwicklung heute auf eine Vielfalt von Alternativen zurückgegriffen werden kann. Im Bereich der oralen Zubereitungen macht sich dabei aber ein Problem immer wieder unangenehm bemerkbar, das bei kosmetischen Anwendungen keine Rolle spielt: Kühlstoffe besitzen häufig unangenehme Geschmacksnoten, die - wenn sie in den Vordergrund treten - eine Verwendung im Bereich von Produkten, die mit der Schleimhaut in Kontakt treten, ausschließen. Typisch werden solche Produkte in unterschiedlichen Kombinationen und verschiedenen Abstufungen mit Attributen wie "stechend", "brennend", "metallisch", "bitter" oder "scharf" belegt. Manche dieser Stoffe sind nachweislich sogar in der Lege, die Mundflora aus dem Gleichgewicht zu bringen.

In der WO 2013/171018 A2 werden Mischungen, enthaltend (a) mindestens ein Phenylalkenal und (b) mindestens eines physiologischen Kühlwirkstoffs beschrieben. Diese Mischungen sollen sich durch eine verbesserte physiologische Kühlwirkung und eine verbesserte geschmackliche Wahrnehmung auszeichnen.

Die EP 1 050 574 A1 betrifft Minz- und/oder Fruchtaromakompositionen, die 8-Ocimenylester enthalten. Die 8-Ocimenylester verstärken in Minz- und/oder Fruchtkompositionen das Geruchs- und Geschmacksempfinden.

Es besteht allerdings nach wie vor großes Interesse an Kühlstoffen, speziell an intelligenten Abmischungen von bekannten Vertretern, die über ein verbessertes Geschmacks- und Leistungsprofil verfügen.

Die Aufgabe der vorliegenden hat daher konkret darin bestanden, die Kühlwirkung bekannter Kühlstoffe durch gezielte Abmischung dahingehend zu verbessert, dass gleichzeitig
- bei oraler Aufnahme eine höhere Kühlwirkung erzielt wird,
- die Kühlwirkung schneller einsetzt,
- die Mundflora in einem gesunden Gleichgewicht gehalten wird,
- ein bitterer Geschmackseindruck, insbesondere in Kombination mit Menthol vermieden oder deutlich vermindert wird,
- der unangenehme Geschmackseindruck, den verschiedene Kühlstoffe für sich aufweisen, abgemildert wird, und
- ein beruhigender Einfluss auf Hals und Stimme ausgeübt wird.

### BESCHREIBUNG DER ERFINDUNG

Ein erster Gegenstand der Erfindung betrifft Kühlstoffmischungen enthaltend
(a) mindestens eine Verbindung der Formel (I) 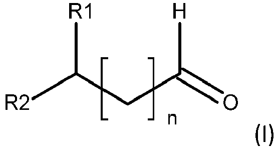 und/oder Isobutyraldehyd,
(b) mindestens eine Verbindung der Formel (II) 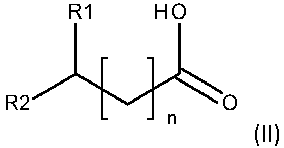 und/oder Isobuttersäure,
   wobei R¹ jeweils für Wasserstoff oder eine Methylgruppe, R² jeweils für eine Methyl-, Ethyl- oder Propylgruppe und n für 1 oder 2 stehen, sowie gegebenenfalls
(c) einen weiteren Kühlstoff,
worin die Mischungen
(a) 90 bis 99 Gew.-% Stoffe der Formel (I) und/oder Isobutyraldehyd,
(b) 1 bis 10 Gew.-% Stoffe der Formel (II) und/oder Isobuttersäure sowie
(c) 0 bis 5 Gew.-% weitere Kühlstoffe
mit der Maßgabe enthalten, dass sich die Komponenten zu 100 Gew.-% ergänzen.

Überraschenderweise wurde gefunden, dass Zubereitungen, die mindestens einen an sich bekannten Kühlstoff aus den Gruppen (a) und (b) enthalten, die eingangs geschilderte komplexe Aufgabe voll umfänglich lösen. Während die Stoffe der Formeln (I) und (II) jeweils für sich genommen einen oftmals unangenehmen stechenden oder sogar brennenden Geschmack auslösen, sind deren Abmischungen deutlich milder und können sogar den Bittergeschmack von Menthol überdecken. Im Gegenteil wirken sie auf Hals und Stimme sogar beruhigend. Ein weiterer Vorteil besteht darin, dass die Kühlwirkung der Mischungen als deutlich stärker und rascher eintretend wahrgenommen wird. Schließlich beeinträchtigen die Mischungen die Zusammensetzung der Mundflora nicht nachteilig. Die geschilderten Effekte treten insbesondere dann besonders deutlich hervor, wenn die Komponente (a) in deutlichem Gewichtsüberschuss zur Komponente (b) eingesetzt wird.

### KÜHLSTOFFE DER GRUPPEN A UND B

Beispiele für Stoffe, die die Komponente (a) bilden, sind Isobutyraldehyd (2220), Isovaleraldehyd (2692) und deren Gemische.

Beispiele für Stoffe, die die Komponente (b) bilden, sind Isobuttersäure (2222), Isovaleriansäure (2694) und deren Gemische.

Die genannten Stoffe stellen bekannte Kühlstoffe dar, die physiologisch unbedenklich und für die orale Verabreichung zugelassen sind. Die oben in Klammern angegebenen Nummern beziehen sich auf die entsprechende GRAS FEMA Registrierung dieser Stoffe.

In einer bevorzugten Ausführungsform enthalten die erfindungsgemäßen Mischungen wenigstens eine der folgenden Mischungen der Komponenten (a) und (b):
(i) Isobutyraldehyd und Isobuttersäure;
(ii) Isovaleraldehyd und Isovaleriansäure;
(iii) Isobutyraldehyd und Isovaleriansäure;
(iv) Isovaleraldehyd und Isobuttersäure;
(v) Isobutyraldehyd, Isobuttersäure, Isovaleraldehyd und Isovaleriansäure.

### WEITERE KÜHLSTOFFE

Als optionale Bestandteile können die erfindungsgemäßen Mischungen weitere Kühlstoffe enthalten, bei denen es sich vorzugsweise um Menthol und/oder Mentholverbindungen handelt.

Kühlstoffe, die im Sinne der Erfindung eingesetzt werden können und die Gruppe (c) bilden, sind - neben dem Grundkörper Menthol selber - beispielsweise ausgewählt aus der Gruppe, die gebildet wird von Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter der Stoffe, die die Komponente (b) bilden, stellt das Monomenthyl Succinate (FEMA GRAS 3810) dar, das als Stoff bereits 1963 von Brown & Williamson Tobacco Corp. patentiert worden **(**US 3,111,127**)** und als Kühlmittel Gegenstand der Schutzrechte US 5,725,865 und 5,843,466 **(V.Mane Fils)** ist. Sowohl das Succinat als auch das analoge Monomenthyl Glutarate (FEMA GRAS 4006) stellen wichtige Vertreter von Monomenthylestern auf Basis von Di- und Polycarbonsäuren dar:

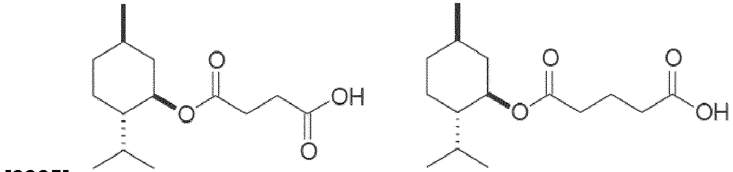

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat® MGC), Menthol Propylenglycol Carbonate (FEMA GRAS 3784 = Frescolat® MPC), Menthol 2-Methyl-1,2-propandiol Carbonate (FEMA GRAS 3849) oder den entsprechenden Zuckerderivaten:

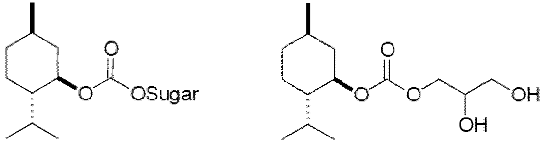

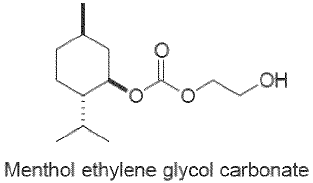

Die Verwendung derartiger Stoffe als Kühlstoff für Zigaretten ist beispielsweise Gegenstand der Druckschrift US 3,419,543 (Mold et al.) aus dem Jahre 1968; die Anwendung als physiologisches Kühlmittel wird in DE 4226043 A1 (H&R) beansprucht.

Im Sinne der Erfindung bevorzugt sind die Mentholverbindungen Menthyl Lactate (FEMA GRAS 3748 = Frescolat® ML) und insbesondere das Menthone Glyceryl Acetal (FEMA GRAS 3807) bzw. Menthone Glyceryl Ketal (FEMA GRAS 3808), das unter der Bezeichnung Frescolat® MGA vermarktet wird.

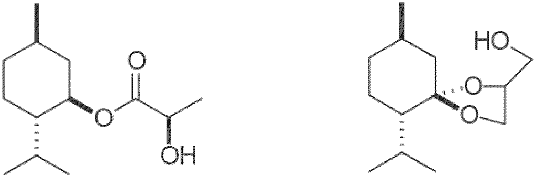

Erstere Struktur wird durch Veresterung von Milchsäure mit Menthol, letztere durch Acetalisierung von Menthon mit Glycerin gewonnen (vgl. DE 2608226 A1**,** H&R). In diese Gruppe von Verbindungen gehört auch das 3-(I-Menthoxy)-1,2,propandiol, das auch als Cooling Agent 10 bekannt ist (FEMA GRAS 3784, vgl. US 6,328,982**,** TIC), sowie das 3-(I-Menthoxy)-2-methyl-1,2,propandiol (FEMA GRAS 3849), das über eine zusätzliche Methylgruppe verfügt.

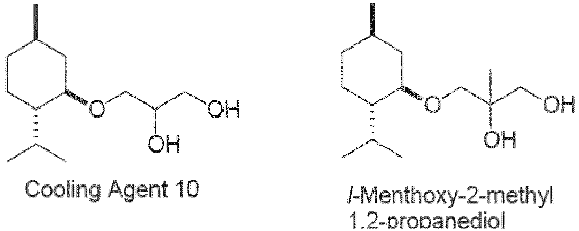

Die Herstellung des 3-(I-Menthoxy)-1,2,propandiol erfolgt beispielsweise ausgehend von Menthol nach dem folgenden Schema (vgl. US 4,459,425**,** Takagaso):

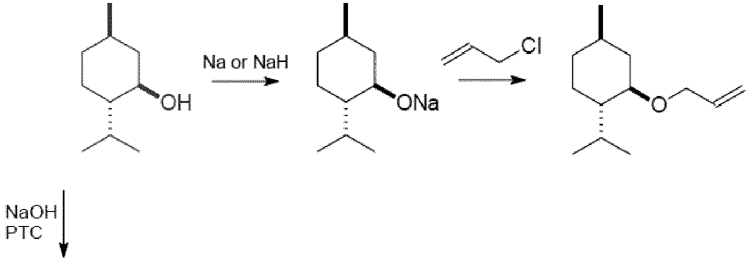

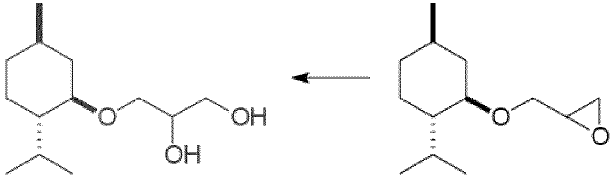

Alternative Routen, bei denen in der ersten Stufe Menthol mit Epichlorhydrin umgesetzt wird, wird in US 6,407,293 und US 6,515,188 (Takagaso) beschrieben. Im Folgenden wird eine Übersicht der bevorzugten Mentholverbindungen gegeben, die sich durch eine CO-Bindung auszeichnen:

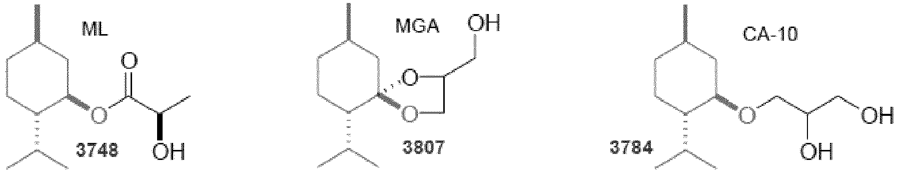

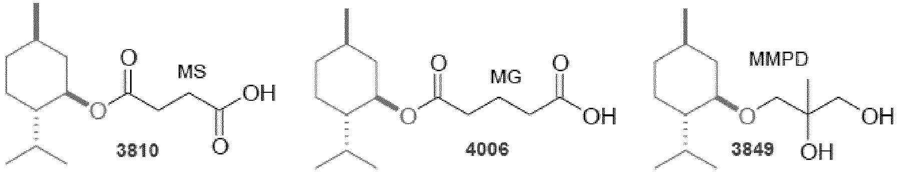

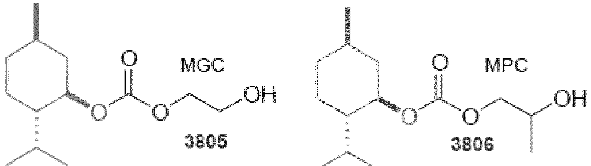

Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat® MGA, Frescolat® ML, Frecolat® MGC und Frescolat® MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern im Sinne der Erfindung eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die ebenfalls im Sinne der Erfindung bevorzugten Spezies WS-3, WS-4, WS-5, WS-12, WS-14 und WS-30. Die beiden nachfolgenden Schaubilder zeigen die Synthesewege auf:

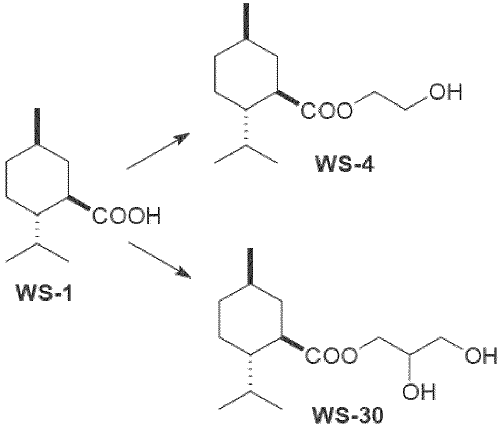

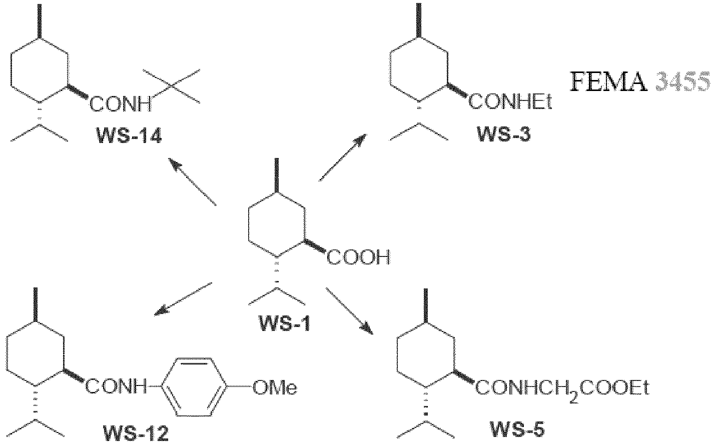

Die sich von WS-1 ableitenden Ester werden beispielsweise in US 4,157,384**,** die entsprechenden N-substituierten Amide in J. Soc. Cosmet. Chem. S. 185-200 (1978**)** beschrieben.

In der vorliegenden Erfindung enthalten die Mischungen die Komponenten in folgenden Mengen:
(a) 90 bis 99 Gew.-% und insbesondere etwa 95 bis etwa 98 Gew.-% Stoffe der Formel (I) und/oder Isobutyraldehyd,
(b) 1 bis 10 Gew.-% und insbesondere etwa 5 bis etwa 8 Gew.-% Stoffe der Formel (II) und/oder Isobuttersäure sowie
(c) 0 bis 5 Gew.-% und insbesondere etwa 1 bis 5 Gew.-% weitere Kühlstoffe mit der Maßgabe, dass sich die Komponenten zu 100 Gew.-% ergänzen.

Mit dieser Einschränkung wird klargestellt, dass alle Mischungsverhältnisse, die sich nicht zu 100 Gew.-% addieren, ausgeschlossen sind und der Fachmann innerhalb der Vorgaben beliebige Mischungsverhältnisse auswählen kann, die die oben geschilderte Lehre erfüllen, ohne hierzu erfinderisch tätig werden zu müssen. Im Übrigen wird auf die Ausführungsbeispiele verwiesen.

### GEWERBLICHE ANWENDBARKEIT

Ein weiterer Gegenstand der vorliegenden Erfindung betrifft Zubereitungen zur oralen Aufnahme, enthaltend die oben beschriebenen Kühlstoffmischungen enthalten. Bei diesen Zubereitungen kann es sich sowohl um Nahrungsmittel, pharmazeutische Zubereitungen oder Mund- und Zahnpflegemittel handeln, wobei der Übergang zwischen diesen Gruppen fließend ist. Ein Kaubonbon kann beispielsweise ebenso als Süßigkeit als auch als Erkältungsmittel Verwendung finden, ein Kaugummi ebenfalls als Erfrischung oder eben zur medizinischen Zahnpflege dienen.

### ORALE ZUBEREITUNGEN

Typische Beispiele für Nahrungsmittel, die die erfindungsgemäßen Zubereitungen enthalten, sind Hartkaramellen, Kaubonbons oder Kaugummis.

Typische Beispiele für pharmazeutische Zubereitungen, die die erfindungsgemäßen Zubereitungen enthalten, sind Erkältungssäfte, Erkältungssprays oder Erkältungsbonbons.

Typische Beispiele für pharmazeutische Zubereitungen, die die erfindungsgemäßen Zubereitungen enthalten, sind Zahnpasten, Mundwässer oder medizinische Kaugummis.

Die genannten oralen Zubereitungen können die Kühlstoffmischungen in Mengen von etwa 0,5 bis etwa 5 Gew.-% und insbesondere etwa 1 bis etwa 2,5 Gew.-% - bezogen auf die Endzubereitung - enthalten. Im Folgenden werden Hilfs- und Zusatzstoffe beschrieben, die die oralen Zubereitungen ebenfalls enthalten können.

### NAHRUNGSMITTEL

Nahrungsmittel, welche die erfindungsgemäßen Kühlstoffmischungen enthalten, stellen in der Regel Hartkaramellen oder Kau- bzw. Lutschbonbons dar. Der wichtigste Zusatzstoff für diese Produkte sind Süßungsmittel, darunter vor allem auch solche, die nicht zuckerbasiert sind. Daneben sind vor allem noch Aromen und Lebensmittelfarbstoffe zu nennen.

### Süßstoffe

Als Süßstoffe oder süß schmeckende Zusatzstoffe kommen zunächst Kohlenhydrate und speziell Zucker in Frage, wie etwa Sucrose/Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

In Betracht kommen auch
synthetische, d.h. in der Regel enzymatisch hergestellte Stärke oder Zuckerhydrolysate (Invertzucker, Fructosesirup);
- Fruchtkonzentrate (z.B. auf Basis von Äpfeln oder Birnen);
- Zuckeralkohole (z.B. Erythritol, Threitol, Arabitol, Ribotol, Xylitol, Sorbitol, Mannitol, Dulcitol, Lactitol);
- Proteine (z.B. Miraculin, Monellin, Thaumatin, Curculin, Brazzein);
- Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
- Süß schmeckende Aminosäuren (z.B. Glycin, D-Leucin, D-Threonin, D-Asparagin, D-Phenylalanin, D-tryptophn, L-Prolin);
- Weitere süß schmeckende niedermolekulare Substanzen, wie z.B. Hernandulcin, Dihydrochalconglykoside, Glycyrrhizin, Glycerrhetinsäure, ihre Derivate und Salze, Extrakte von Lakritz (Glycyrrhizza glabra ssp.), Lippia dulcis Extrakte, Momordica ssp. Extrakte oder
- Einzelsubstanzen wie z.B. Momordica grosvenori [Luo Han Guo] und die daraus gewonnenen Mogroside, Hydrangea dulcis oder Stevia ssp. (z.B. Stevia rebaudiana) Extrakte.

### Aromastoffe

Die Erfindung erlaubt in den oralen Zubereitungen insbesondere auch den Einsatz von Aromastoffen mit Ester-, Aldehyd- oder Lactonstruktur, die in Gegenwart von Titandioxid und unter Lichteinfluss besonders schnell abgebaut werden. Die Erfindung sorgt somit auch für eine verbesserte Stabilität, speziell Lagerstabilität der Aromastoffe.

Die erfindungsgemäßen oralen Zubereitungen können einen oder mehrere Aromastoffe enthalten. Typische Beispiele umfassen: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-lsopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

### Geschmacksverstärker

Die oralen Zubereitungen- wie auch die Aromamischungen- können des Weiteren zusätzliche Aromastoffe zum Verstärken eines salzigen, gegebenenfalls leicht sauren und/oder Umami-Geschmackseindrucks enthalten. Es werden somit die erfindungsgemäßen Produkte bzw. Aromamischungen in Kombination mit zumindest einer weiteren zur Verstärkung eines an-genehmen Geschmackseindrucks (salzig, Umami, gegebenenfalls leicht sauer) geeigneten Substanz verwendet. Hierbei bevorzugt sind salzig schmeckende Verbindungen und salzver-stärkende Verbindungen. Bevorzugte Verbindungen sind in der WO 2007/045566 offenbart. Ferner bevorzugt sind Umami-Verbindungen wie in der WO 2008/046895 und EP 1 989 944 beschrieben sind.

Weiterhin können erfindungsgemäß bevorzugte Produkte auch Aromastoffe zur Maskierung von bitteren und/oder adstringierenden Geschmackseindrücken umfassen (Geschmackskorrigentien). Die (weiteren) Geschmackskorrigenzien werden z. B. aus der folgenden Liste ausgewählt: Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), weitere Hydroxyflavanone (z.B. Eriodictyol, Homoeriodictyol oder deren Natriumsalze), insbesondere gemäß US 2002/0188019, Hydroxybenzoesäureamide nach DE 10 2004 041 496 (z.B. 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid, 4-Hydroxybenzoesäure-N-(4-hydroxy-3-methoxybenzyl)amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)-ethylamid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid (Aduncamid), 4-Hydroxybenzoesäurevanillylamid), bittermaskierende Hydroxydeoxybenzoine z.B. gemäß WO 2006/106023 (z.B. 2-(4-Hydroxy-3-methoxyphenyl)-1-(2,4,6-tri-hydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-meth-oxy-phenyl)ethanon), Aminosäuren (z.B. gamma-Aminobuttersäure nach WO 2005/096841 zur Verminderung oder Maskierung eines unangenehmen Geschmackseindrucks wie Bitterkeit), Äpfelsäureglycoside nach WO 2006/003107, salzig schmeckende Mischungen gemäß PCT/EP 2006/067120 Diacetyltrimere gemäß WO 2006/058893, Gemische von Molkeproteinen mit Lecithinen und/oder bittermaskierende Substanzen wie Gingerdione gemäß WO 2007/003527.

Bevorzugte Aromastoffe sind solche, die einen süßen Geruchseindruck verursachen, wobei der oder die weiteren Aromastoffe, die einen süßen Geruchseindruck verursachen, bevor-zugt ausgewählt sind aus der Gruppe bestehend aus:

Vanillin, Ethylvanillin, Ethylvanillinisobutyrat (= 3 Ethoxy-4-isobutyryloxy-benzaldehyd), Furaneol (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalac-ton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäurei-soamylester, 3-Methyl-buttersäureethylester, n-Hexansäureethylester, n-Hexansäure-allylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al, 4-Hydroxyzimtsäure, 4-Methoxy-3-hydroxyzimtsäure, 3-Methoxy-4-hydroxyzimtsäure, 2-Hydroxyzimtsäure, 2,4-Dihydroxybenzoesäure, 3-Hydroxybenzoesäure, 3,4-Dihydroxybenzoesäure, Vanillinsäure, Homovanillinsäure, Vanillomandelsäure und Phenylacetaldehyd.

### Wirkstoffe zur Maskierung von unangenehmen Geschmackseindrücken

Weiterhin können die oralen Zubereitungen auch weitere Stoffe umfassen, die ebenfalls zur Maskierung von bitteren und/oder adstringierenden Geschmackseindrücken dienen. Diese weiteren Geschmackskorrigenzien werden z. B. aus der folgenden Liste ausgewählt: Nucleotiden (z.B. Adenosin-5'-monophosphat, Cytidin-5'-monophosphat) oder deren physiologisch akzeptablen Salzen, Lactisolen, Natriumsalzen (z.B. Natriumchlorid, Natriumlactat, Natriumcitrat, Natriumacetat, Natriumgluconoat), Hydroxyflavanonen, dabei bevorzugt Eriodictyol, Sterubin (Eriodictyol-7-methylether), Homoeriodictyol, und deren Natrium-, Kalium-, Calcium-, Magnesium- oder Zinksalzen (insbesondere solche wie beschrieben in EP 1258200 A2, die bezüglich der darin offenbarten entsprechenden Verbindungen auf dem Wege der Verweisung Bestandteil dieser Anmeldung wird), Hydroxybenzoesäureamiden, dabei vorzugsweise 2,4-Dihydroxybenzoesäurevanillylamid, 2,4-Dihydroxybenzoesäure-*N-*(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N*-(4-hydroxy-3-methoxybenzyl)amid, 2,4-Dihydroxybenzoesäure-*N*-(4-hydroxy-3-methoxy-benzyl)amid-Mono-natriumsalz, 2,4-Dihydroxybenzoesäure-*N*-2-(4-hydroxy-3-methoxy-phenyl)ethylamid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-*N*-[2-(4-hydroxy-3-methoxyphenyl)ethyl]amid; 4-Hydroxybenzoesäurevanillylamiden (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, dabei vorzugsweise 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)-ethanon und 1-(2-Hydroxy-4-methoxyphenyl)-2-(4-hydroxy-3-methoxyphenyl)ethanon) (insbesondere solche wie beschrieben in WO 2006/106023 beschrieben, die bezüglich der darin offenbarten entsprechenden Verbindungen auf dem Wege der Verweisung Bestandteil dieser Anmeldung wird); Hydroxyphenylalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); gamma-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird); Divanillinen (insbesondere solche wie beschrieben in WO 2004/078302, die bezüglich der darin offenbarten entsprechenden Verbindungen auf dem Wege der Verweisung Bestandteil dieser Anmeldung wird) und 4-Hydroxydihydrochalconen (vorzugsweise wie beschrieben in US 2008/0227867 A1, die bezüglich der darin offenbarten entsprechenden Verbindungen auf dem Wege der Verweisung Bestandteil dieser Anmeldung wird), dabei insbesondere Phloretin und Davidigenin, Aminosäuren oder Gemische von Molkeproteinen mit Lecithinen, Hesperetin wie in der WO 2007/014879 offenbart, die hinsichtlich dieser Verbindungen auf dem Wege der Verweisung Bestandteil dieser Anmeldung wird, 4-Hydroxydihydrochalkonen wie in der WO 2007/107596 offenbart, die hinsichtlich dieser Verbindungen auf dem Wege der Verweisung Bestandteil dieser Anmeldung wird, oder Propenylphenylglycosiden (Chavicolglycosiden) wie in EP 1955601 A1 beschrieben, die hinsichtlich dieser Verbindungen auf dem Wege der Verweisung Bestandteil dieser Anmeldung wird, oder Extrakten aus *Rubus suavissimus,* Extrakte aus *Hydrangea macrophylla* wie in EP 2298084 A1 beschrieben, Pellitorin und abgeleiteten Aromakompositionen wie in EP 2008530 A1 beschrieben, Umami-Verbindungen wie in WO 2008/046895 A1 und EP 1989944 A1 beschrieben, Umami-Verbindungen wie beschrieben in EP 2064959 A1 bzw. EP 2135516 A1, Vanillyllignanen, Enterodiol, sowie N-Decadienoylaminosäuren und deren Gemische.

### Lebensmittelfarbstoffe

Lebensmittelfarbstoffe oder kurz Farbstoffe sind Lebensmittelzusatzstoffe zum Färben von Lebensmittel. Farbstoffe werden in die Gruppen der natürlichen Farbstoffe und synthetischen Farbstoffe unterteilt. Die naturidentischen Farbstoffe sind ebenfalls synthetischen Ursprungs. Die naturidentischen Farbstoffe sind synthetische Nachbildungen von in der Natur vorkommenden, färbenden Substanzen. Geeignete Farbstoffe für den Einsatz in der vorliegenden Zusammensetzung sind ausgewählt aus: Kurkumin, E 100 Riboflavin, Lactoflavin, Laktoflavin, Vitamin B2, E 101 Tartrazin, E 102 Chinolingelb, E 104 Gelborange S, Gelborange RGL, E 110 Cochenille, Karminsäure, echtes Karmin, E 120 Azorubin, Carmoisin, E 122 Amaranth, E 123 Cochenillerot A, Ponceau 4 R, Victoriascharlach 4 R, E 124 Erythrosin, E 127 Allurarot AC, E 129 Patentblau V, E 131 Indigotin, Indigo-Karmin, E 132 Brillantblau FCF, Patentblau AE, Amidoblau AE, E 133 Chlorophylle, Chlorophylline, E 140 Kupferkomplexe der Chlorophylle, Kupfer-Chlorophyllin-Komple, E 141 Brillantsäuregrün, Grün S, E 142 Zuckerkulör, Zuckercouleur, E 150 a Sulfitlaugen-Zuckerkulör, E 150 b Ammoniak-Zuckerkulör, E 150 c Ammoniumsulfit-Zuckerkulör, E 150 d Brillantschwarz FCF, Brillantschwarz PN, Schwarz PN, E 151 Pflanzenkohle, E 153 Braun FK, E 154 Braun HT, E 155 Carotin, Karotin, E 160 a Annatto, Bixin, Norbixin, E 160 b Capsanthin, Capsorubin, E 160 c Lycopin, E 160 d Beta-apo-8'-Carotinal, Apocarotinal, Beta-Apocarotinal, E 160 e Beta-apo-8'-Carotinsäure-Ethylester (C30), Apocarotinester, Beta-Carotinsäureester, E 160 f Lutein, Xanthophyll, E 161 b Canthaxanthin, E 161 g Betanin, Betenrot, E 162 Anthocyane, E 163 Calciumcarbonat, E 170 Titandioxid, E 171 Eisenoxide, Eisenhydroxide, E 172 Aluminium, E 173 Silber, E 174 Gold, E 175 Litholrubin BK, Rubinpigment BK, E 180.

### KAUGUMMIS

Bei den bevorzugten oralen Zubereitungen kann es sich insbesondere auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500**,** auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### MUND- UND ZAHNPFLEGEMITTEL

Erfindungsgemäße oral konsumierbare süß schmeckende Produkte können insbesonere der Mund- und Zahnreinigung und-pflege dienen. Beispiele hierfür sind wie gesagt Zahnpasten, Zahngele, Zahnpulver, Mundwässer und dergleichen. Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummi arabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam®, (L-Aspartyl- L-phenylalanin-methylester), Stiviaextrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der oralen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### VERFAHREN UND VERWENDUNG

Ein weiterer Gegenstand der Erfindung betrifft ein Verfahren zur Verstärkung des kühlenden Effektes, den eine Zubereitung bei oraler Aufnahme bewirkt, wobei man der Zubereitung eine der oben beschriebenen erfindungsgemäßen Kühlstoffmischungen, vorzugsweise in Mengen von etwa 0,005 bis etwa 5 Gew.-%, vorzugsweise von etwa 0,05 bis etwa 1 Gew.-% und insbesondere von etwa 0,01 bis etwa 0,5 Gew.-% zufügt.

Ein letzter Gegenstand der Erfindung betrifft die Verwendung von Mischungen nach Anspruch 1 als Kühlstoffe und zwar wiederum vorzugsweise in Mengen von etwa 0,005 bis etwa 5 Gew.-%, vorzugsweise von etwa 0,05 bis etwa 1 Gew.-% und insbesondere von etwa 0,01 bis etwa 0,5 Gew.-% zufügt.

Soweit Verfahren und Verwendung wie oben erläutert betroffen sind, gelten die gleichen Mengenangaben und bevorzugten Ausführungsformen wie vorstehend ausführlich beschrieben mit, ohne dass es hierzu einer Wiederholung bedarf.

### BEISPIELE

### ANWENDUNGSTECHNISCHE UNTERSUCHUNGEN

### BEISPIELE 1 BIS 6, VERGLEICHSBEISPIELE V1 BIS V4

Die Kühlwirkung und geschmackliches Profil unterschiedlicher Kühlstoffmischungen wurden von einem Panel bestehend aus 5 geschulten Testern beurteilt. Dazu wurden die Mischungen in eine ungezuckerte und nicht aromatisierte Standard-Kaubonbonzubereitung eingearbeitet. Die Ergebnisse sind in Tabelle 1 wiedergegeben. Die Bewertung erfolgte gemäß der Benotung (5) = trifft sehr zu bis (0) = trifft weitgehend nicht zu. Angegeben sind die Mittelwerte. Die Beispiele 1 bis 6 sind erfindungsgemäß, die Beispiele V1 bis V4 dienen zum Vergleich.

**Tabelle 1**

| Sensorische Beurteilung von Kühlstoffmischungen | | | | | | | | | | |
|---|---|---|---|---|---|---|---|---|---|---|
| **Komponenten** | **V1** | **V2** | **V3** | **V4** | **1** | **2** | **3** | **4** | **5** | **6** |
| Isobutyraldehyd | 100 | - | - | - | 99 | 99 | | - | 45 | 0,5 |
| Isovaleraldehyd | - | 100 | - | - | - | - | 99 | 99 | 45 | |
| Isobuttersäure | - | - | 100 | - | 1 | - | 1 | - | 5 | 0,5 |
| Isovaleriansäure | - | - | - | 100 | - | 1 | - | 1 | 5 | |
| Menthol | - | - | - | - | - | - | - | - | - | 10 |
| Menthylacetat | - | - | | - | - | - | - | - | - | 89 |

| Sensorische Beurteilung | | | | | | | | | | |
|---|---|---|---|---|---|---|---|---|---|---|
| Kühlwirkung ist stark | 3,2 | 3,1 | 3,0 | 3,1 | 4,2 | 4,4 | 4,1 | 4,4 | 4,6 | 4,9 |
| Kühlwirkung tritt schnell ein | 2,1 | 2.0 | 2,2 | 2,5 | 3,8 | 3,5 | 3,7 | 3,6 | 4,1 | 4,4 |
| Brennender Geschmack | 4,0 | 4,0 | 4,0 | 4,0 | 2,3 | 2,5 | 2,9 | 2,8 | 2,0 | 3,0 |
| Stechender Geschmack | 4,0 | 4,0 | 3,9 | 3,8 | 2,5 | 2,1 | 2,1 | 2,4 | 2,3 | 2,8 |
| Bitterer Geschmack | 2,0 | 2,0 | 2,0 | 1,8 | 2,1 | 2,1 | 2,0 | 2,0 | 1,8 | 2,0 |

Die Beispiele und Vergleichsbeispiele zeigen, dass die erfindungsgemäßen Mischungen eine höhere und raschere Kühlwirkung entfalten und dabei sensorisch deutlich besser als die Einzelsubstanzen beurteilt werden.

Im Folgenden werden eine Reihe von Formulierungsbeispielen gegeben.

### FORMULIERUNGSBEISPIELE

### Beispiel 1

Zahnpasta (Mengenangaben als Gew.-%)

| **KOMPONENTEN** | **MENGE** |
|---|---|
| Water (deionized) | Ad 100 |
| Sorbitol 70% | 45.00 |
| Trisodiumphosphate | 0.10 |
| Saccharin | 0.20 |
| Sodium monofluorophosphate | 1.14 |
| PEG 1500 | 5.00 |
| Sident 9 (abrasive silica) | 10.00 |
| Sident 22 S (Thickening silica) | 8.00 |
| Sodiumcarboxymethylcellulose | 1.10 |
| Titanium (IV) oxide | 0.50 |
| Water (deionized) | 4.50 |
| Sodiumlaurylsulfate (SLS) | 1.50 |
| Aromamischung | 1.00 |
| Solbrol M (Sodium salt) (Methylparaben) | 0.15 |
| Kühlstoffmischung I | 0.40 |

### Beispiel 2

Zahnpasta mit Zinkcitrat (Mengenangaben als Gew.-%)

| **KOMPONENTEN** | **MENGE** |
|---|---|
| Water (deionized) | Ad 100 |
| Sorbitol 70% | 45.00 |
| Trisodiumphosphate | 0.10 |
| Saccharin | 0.20 |
| Sodium monofluorophosphate | 1.14 |
| PEG 1500 | 5.00 |
| Sident 9 (abrasive silica) | 10.00 |
| Sident 22 S (Thickening silica) | 8.00 |
| Sodiumcarboxymethylcellulose | 1.10 |
| Zinc citrate | 1.00 |
| Titanium (IV) oxide | 0.50 |
| Water (deionized) | 4.50 |
| Sodiumlaurylsulfate (SLS) | 1.50 |
| Aromamischung | 1.00 |
| SymDiol® 68 (1.2-Hexanediol. Caprylylglycol) | 0.25 |
| Kühlstoffmischung II | 0.10 |

### Beispiel 3

Mundwasser (Mengenangabe als Gew.-%)

| **KOMPONENTEN** | **MENGE** |
|---|---|
| Ethylalcohol | 10.00 |
| Cremophor CO 40 (PEG 40 hydrogenated castor oil) | 1.00 |
| Aromamischung | 0.25 |
| Water (deionized) | To 100.00 |
| Sorbitol 70% | 5.00 |
| Sodiumsaccharin 450 | 0.07 |
| Sodiumfluoride | 0.18 |
| Benzoic acid | 0.12 |
| Kühlstoffmischung III | 0.30 |

### Beispiel 4

Dentalgel (Mengenangaben als Gew.-%)

| **KOMPONENTEN** | **MENGE** |
|---|---|
| Na carboxymethylcellulose | 0.40 |
| Sorbitol 70 %. in water | 72.00 |
| Polyethylene glycol (PEG) 1500 | 3.00 |
| Na saccarinate | 0.07 |
| Na fluoride | 0.24 |
| Aromamischung | 1.00 |
| Abrasive silica | 11.00 |
| Thickening silica | 6.00 |
| Sodium dodecyl sulfate (SDS) | 1.40 |
| Dist. water | Ad 100 |
| p-Hydroxybenzoic acid (PHB) ethyl ester | 0.15 |
| Kühlstoffmischung IV | 0.20 |

### Beispiel 5

Anti-Plaque Zahncreme (Mengenangaben als Gew.-%)

| **KOMPONENTEN** | **MENGE** |
|---|---|
| Carrageenan | 0.90 |
| Glycerol | 15.00 |
| Sorbitol 70 %. in water | 25.00 |
| PEG 1000 | 3.00 |
| Na fluoride | 0.24 |
| Tetrapotassium diphosphate | 4.50 |
| Tetrasodium diphosphate | 1.50 |
| Na saccarinate | 0.40 |
| Precipitated silica | 20.00 |
| Titanium dioxide | 1.00 |
| Triclosan | 0.30 |
| Spearmint flavor (comprising 60 wt.% I-carvone and 25 wt.% I-menthol) | 1.00 |
| Sodium dodecyl sulfate | 1.30 |
| Dist. water | Ad 100 |
| Benzylalcohol | 0.50 |
| Kühlstoffmischung V | 0.25 |

### Beispiel 6

Zahncreme für empfindliche Zähne (Mengenangaben als Gew.-%)

| **KOMPONENTEN** | **MENGE** |
|---|---|
| Na carboxymethylcellulose | 0.70 |
| Xanthan gum | 0.50 |
| Glycerol | 15.00 |
| Sorbitol 70 %. in water | 12.00 |
| Potassium nitrate | 5.00 |
| Sodium monofluorophosphate | 0.80 |
| Na saccharinate | 0.20 |
| Aromamischung | 1.00 |
| Ca-carbonate | 35.00 |
| Silicon dioxide | 1.00 |
| Sodium dodecyl sulfate (SDS) | 1.50 |
| Dist. water | Ad 100 |
| PHB methyl ester and PHB propyl ester | 0.20 |
| Kühlstoffmischung I | 0.50 |

### Beispiel 7

Zahncreme und Mundwasser 2:1 (Mengenangaben als Gew.-%)

| **KOMPONENTEN** | **MENGE** |
|---|---|
| Sorbitol | 40.00 |
| Glycerol | 20.00 |
| Ethanol | 5.00 |
| Water | Ad 100 |
| Na monofluorophosphate | 0.75 |
| Saccharin | 0.20 |
| Sident 9 (abrasive silicon dioxide) | 20.00 |
| Sident 22 S (thickening silicon dioxide) | 2.00 |
| Sodium carboxymethylcellulose | 0.30 |
| Sodium lauryl sulfate (SDS) | 1.20 |
| Color (Suspension. 1% in water) C.I. Pigment Blue 15 | 0.50 |
| Aromamischung | 0.90 |
| Solbrol M. sodium salt (methylparaben. sodium salt) | 0.20 |
| Kühlstoffmischung II | 0.30 |

### Beispiel 8

Mundwasser mit Fluorid (Mengenangaben als Gew.-%)

| **KOMPONENTEN** | **MENGE** |
|---|---|
| Ethanol | 7.00 |
| Glycerol | 12.00 |
| Na fluoride | 0.05 |
| Pluronic F-127® (BASF. surface-active substance) | 1.40 |
| Na phosphate buffer pH 7.0 | 1.10 |
| Na saccharinate | 0.10 |
| Aromamischung | 0.15 |
| Chlorhexidine digluconate | 0.2 |
| Dist. water | to 100 |
| Sorbic acid | 0.20 |
| Kühlstoffmischung III | 0.30 |

### Beispiel 9

Zuckerfreies Kaugummi (Mengenangaben als Gew.-%)

| **KOMPONENTEN** | **MENGE** |
|---|---|
| Chewing gum base | 30.00 |
| Sorbitol. powder | Ad 100 |
| Palatinite | 9.50 |
| Xylitol | 2.00 |
| Mannitol | 3.00 |
| Aspartame | 0.10 |
| Acesulfame K | 0.10 |
| Emulgum / emulsifier | 0.30 |
| Sorbitol 70 %. in water | 14.00 |
| Glycerol | 1.00 |
| Aromamischung | 1.50 |
| Kühlstoffmischung IV | 0.20 |

## Patentansprüche

1. Kühlstoffmischungen enthaltend
(a) mindestens eine Verbindung der Formel (I) 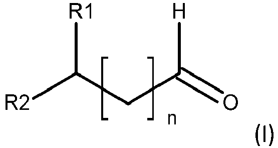 und/oder Isobutyraldehyd,
(b) mindestens eine Verbindung der Formel (II) 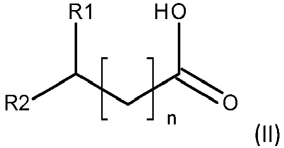 und/oder Isobuttersäure,
wobei R¹ jeweils für Wasserstoff oder eine Methylgruppe, R² jeweils für eine Methyl-, Ethyl- oder Propylgruppe und n für 1 oder 2 stehen, sowie gegebenenfalls
(c) einen weiteren Kühlstoff,
worin die Mischungen
(a) 90 bis 99 Gew.-% Stoffe der Formel (I) und/oder Isobutyraldehyd,
(b) 1 bis 10 Gew.-% Stoffe der Formel (II) und/oder Isobuttersäure sowie
(c) 0 bis 5 Gew.-% weitere Kühlstoffe
mit der Maßgabe enthalten, dass sich die Komponenten zu 100 Gew.-% ergänzen.

2. Mischungen nach Anspruch 1, **dadurch gekennzeichnet, dass** sie als Komponente (a) Isobutyraldehyd oder Isovaleraldehyd oder deren Gemische enthalten.

3. Mischungen nach den Ansprüchen 1 und/oder 2, **dadurch gekennzeichnet, dass** sie als Komponente (b) Isobuttersäure oder Isovaleriansäure oder deren Gemische enthalten.

4. Mischungen nach mindestens einem der Ansprüche 1 bis 3, **dadurch gekennzeichnet, dass** sie als Komponente (c) Menthol oder Menthylacetat oder deren Gemische enthalten.

5. Mischungen nach mindestens einem der Ansprüche 1 bis 4, **dadurch gekennzeichnet, dass** sie wenigstens eine der folgenden Mischungen der Komponenten (a) und (b) enthalten:
(i) Isobutyraldehyd und Isobuttersäure;
(ii) Isovaleraldehyd und Isovaleriansäure;
(iii) Isobutyraldehyd und Isovaleriansäure;
(iv) Isovaleraldehyd und Isobuttersäure;
(v) Isobutyraldehyd, Isobuttersäure, Isovaleraldehyd und Isovaleriansäure.

6. Zubereitungen zur oralen Aufnahme, enthaltend die Mischungen nach Anspruch 1.

7. Zubereitungen nach Anspruch 6, **dadurch gekennzeichnet, dass** es sich um Nahrungsmittel, pharmazeutische Zubereitungen und/oder Mund- und Zahnpflegemittel handelt.

8. Zubereitungen nach Anspruch 7, **dadurch gekennzeichnet, dass** es sich um Hartkaramellen, Kaubonbons oder Kaugummis handelt.

9. Zubereitungen nach Anspruch 7, **dadurch gekennzeichnet, dass** es sich um Erkältungssäfte, Erkältungssprays oder Erkältungsbonbons handelt.

10. Zubereitungen nach Anspruch 7, **dadurch gekennzeichnet, dass** es sich um Zahnpasten, Mundwässer oder medizinische Kaugummis handelt.

11. Zubereitungen nach mindestens einem der 6 bis 10, **dadurch gekennzeichnet, dass** sie die Mischungen nach Anspruch 1 in Mengen von 0,005 bis 5 Gew.-% - bezogen auf die Endzubereitung - enthalten.

12. Verfahren zur Verstärkung des kühlenden Effektes, den eine Zubereitung bei oraler Aufnahme bewirkt, **dadurch gekennzeichnet, dass** man der Zubereitung eine Mischung gemäß Anspruch 1 zufügt.

13. Verwendung von Mischungen nach Anspruch 1 als Kühlstoffe.

## Claims

1. Coolant mixtures comprising
(a) at least one compound of the formula (I) 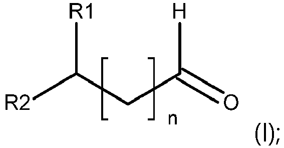 and/or isobutyraldehyde
(b) at least one compound of the formula (II) 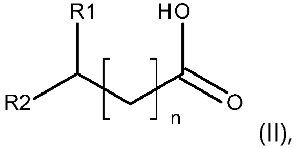 and/or isobutyric acid
wherein R¹ is in each case hydrogen or a methyl group, R² is in each case a methyl, ethyl or propyl group and n is_1, or 2; and optionally
(c) a further coolant,
wherein said mixture comprises:
(a) 90 to about 99% by weight substances of the formula (I) and/or isobutyraldehyde,
(b) 1 to 10% by weight substances of the formula (II) and/or isobutyric acid, and
(c) 0 to 5% by weight further coolants
with the proviso that the components add up to 100% by weight.

2. Mixtures according to Claim 1, wherein, said mixture comprise isobutyraldehyde or isovaleraldehyde or mixtures thereof as component (a).

3. Mixtures according to claims 1 and/or 2, wherein said mixtures comprise isobutyric acid or isovaleric acid or mixtures thereof as component (b).

4. Mixtures according to at least one of claims 1 to 3, wherein said mixtures comprise one selected from menthol, menthyl acetate, and mixtures thereof as component (c).

5. Mixtures according to at least one of claims 1 to 4, wherein said mixtures comprise at least one mixture of components (a) and (b) selected from:
(i) isobutyraldehyde and isobutyric acid;
(ii) isovaleraldehyde and isovaleric acid;
(iii) isobutyraldehyde and isovaleric acid;
(iv) isovaleraldehyde and isobutyric acid; and
(v) isobutyraldehyde, isobutyric acid, isovaleraldehyde and isovaleric acid.

6. Preparations for oral administration comprising the mixtures according to claim 1.

7. The preparation according to claim 6, wherein said preparation takes a form of one selected from foodstuffs, pharmaceutical preparations and/or mouth compositions and dental care compositions.

8. The preparation, according to Claim 7, wherein said preparation takes a form of one selected from hard candies, chewy candies, and chewing gums.

9. The preparation, according to Claim 8, wherein said preparation takes a form of a cold remedy.

10. The preparation, according to Claim 9, wherein said preparation takes a form of one selected from toothpastes, mouthwashes, and medicinal chewing gums.

11. Preparations according to at least one of claims 6 to 10, wherein said preparations comprise the mixtures according to claim 1 in an amount of from 0.005 to 5% by weight - based on the finished preparation.

12. A method for enhancing the cooling effect caused by a preparation on oral administration, the method comprising adding the mixture, according to Claim 1, to the preparation.

13. Uses of mixtures according to claim 1 as coolants.

## Revendications

1. Mélanges d'agents rafraîchissants contenant
(a) au moins un composé de formule (I) 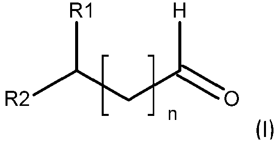 et/ou de l'isobutyraldéhyde,
(b) au moins un composé de formule (II) 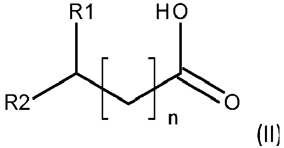 et/ou de l'acide isobutyrique,
où R¹ représente à chaque fois hydrogène ou un groupe méthyle, R² représente à chaque fois un groupe méthyle, un groupe éthyle ou un groupe propyle et n vaut 1 ou 2, ainsi que le cas échéant
(c) un autre agent rafraîchissant,
où les mélanges contiennent
(a) 90 à 99% en poids de substances de formule (I) et/ou d'isobutyraldéhyde,
(b) 1 à 10% en poids de substances de formule (II) et/ou d'acide isobutyrique,
(c) 0 à 30% en poids d'autres agents rafraîchissants
à condition que la somme des composants vaille 100% en poids.

2. Mélanges selon la revendication 1, **caractérisés en ce qu'**ils contiennent, comme composant (a), de l'isobutyraldéhyde ou de l'isovaléraldéhyde ou leurs mélanges.

3. Mélanges selon les revendications 1 et/ou 2, **caractérisés en ce qu'**ils contiennent, comme composant (b), de l'acide isobutyrique ou de l'acide isovalérianique ou leurs mélanges.

4. Mélanges selon au moins l'une quelconque des revendications 1 à 3, **caractérisés en ce qu'**ils contiennent, comme composant (c), du menthol ou de l'acétate de menthyle ou leurs mélanges.

5. Mélanges selon au moins l'une quelconque des revendications 1 à 4, **caractérisés en ce qu'**ils contiennent au moins un des mélanges suivants des composants (a) et (b) :
(i) isobutyraldéhyde et acide isobutyrique ;
(ii) isovaléraldéhyde et acide isovalérianique ;
(iii) isobutyraldéhyde et acide isovalérianique ;
(iv) isovaléraldéhyde et acide isobutyrique ;
(v) isobutyraldéhyde, acide isobutyrique, isovaléraldéhyde et acide isovalérianique.

6. Préparations pour la prise par voie orale, contenant les mélanges selon la revendication 1.

7. Préparations selon la revendication 6, **caractérisées en ce qu'**il s'agit d'aliments, de préparations pharmaceutiques et/ou d'agents de soin de la bouche et des dents.

8. Préparations selon la revendication 7, **caractérisées en ce qu'**il s'agit de caramels durs, de bonbons à mâcher ou de gommes à mâcher.

9. Préparations selon la revendication 7, **caractérisées en ce qu'**il s'agit de jus rafraîchissants, de sprays rafraîchissants ou de bonbons rafraîchissants.

10. Préparations selon la revendication 7, **caractérisées en ce qu'**il s'agit de dentifrices, de bains de bouche ou de gommes à mâcher médicales.

11. Préparations selon au moins l'une quelconque des revendications 6 à 10, **caractérisées en ce qu'**elles contiennent les mélanges selon la revendication 1 en des quantités de 0,005 à 5% en poids - par rapport à la préparation finale.

12. Procédé pour le renforcement de l'effet rafraîchissant provoqué par une préparation lors de la prise par voie orale, **caractérisé en ce qu'**on ajoute un mélange selon la revendication 1 à la préparation.

13. Utilisation de mélanges selon la revendication 1 comme agents rafraîchissants.
